# EUROPEAN PATENT APPLICATION

(11) **EP 4 477 174 A1**
(43) Date of publication of application: **18.12.2024**
(21) Application number: 23315250.3
(22) Date of filing: 16.06.2023
(51) Int. Cl.: A61B 34/10

(54) **SYSTEM, METHOD, COMPUTER PROGRAM AND COMPUTER PROGRAM PRODUCT FOR AUTOMATIC PLANNING OF A CLINICAL INTERVENTION FOR IMPLANTING A CARDIAC VALVE**

(71) Applicant: Caranx Medical, 75013 Paris (FR)
(72) Inventor: MIRA, Anna, 06000 Nice (FR); CERRUTI, Giulio, 06700 Saint-Laurent-Du-Var (FR); BECHAR, Ikhlef, 06410 Biot (FR); PRZYBYLSKI, Flavie, 06800 Cagnes sur Mer (FR); BERTHET-RAYNE, Pierre, 06800 Cagnes-sur-Mer (FR); SMITS, Jonas Victor Harmen, 3360 Bierbeek (BE); TCHETCHE, Didier, 31000 Toulouse (FR)
(74) Representative: Hepp Wenger Ryffel AG

(57) **Abstract**

The present invention relates to a system, a method, a computer program and a computer program product for automatic planning of a clinical intervention for implanting a cardiac valve in a patient.

The system (100) comprises an input interface (101) for entering data and a computing unit (102). Said computing unit (102) is adapted to receive input data relating to characteristics of the patient via the input interface 101), to automatically select a surgical tool (VT, DT) and a surgical method characteristic (AS, VP, PS) based on the characteristics of the patient, the surgical tool (VT, DT) and the surgical method characteristic (AS, VP, PS) being suitable for implanting the cardiac valve in the patient, and to provide output data relating at least to the selected surgical tool (VT, DT) and the selected surgical method characteristic (AS, VP, PS).

## Description

The present invention relates to a system, a method, a computer program and a computer program product for automatic planning of a clinical intervention for implanting a cardiac valve in a patient according to the independent claims.

Implanting a cardiac valve may concern an aortic valve replacement, a mitral replacement, a tricuspid valve replacement or a pulmonary valve replacement by an artificial heart valve. Current aortic valve replacement approaches include closed heart surgery. Trans-catheter aortic valve implantation (TAVI) or trans-catheter aortic valve replacement (TAVR) is a minimally invasive procedure for treating a failing aortic valve. A valve implant (e.g. a bioprosthetic valve made of e.g. porcine pericardium sutured on a metal stent) is crimped inside a catheter. The catheter is inserted, for example, via the femoral artery and is pushed upstream along the aorta up to the diseased native valve, within which the valve implant is deployed. Main complications during implantation are vascular injury, stroke, cardiac injury (heart block, coronary obstruction, cardiac perforation), aortic regurgitation, cardiac conduction abnormalities and valve misplacement. Typically, the implantation process is image guided and makes use of patient images taken and annotated in the planning stage. Identifying how best to perform the procedure is time consuming, requires considerable skills of the operator and bears the risk of errors.

The size of the valve implant is relevant for the outcome of the surgical procedure. Undersizing of a valve implant may lead to paravalvular aortic regurgitation, while oversizing may result in a rupture of the aortic annulus or in a suboptimal functional behaviour of the implant.

WO2019/179793 A1 discloses methods and systems for automatically selecting a cardiac implant from a series of cardiac implants and a method for estimating a risk of complications.

WO2006/092600 A1 concerns automatically planning at least a part of a surgical procedure, wherein a model is instantiated, and surgical planning information adapted for the patient's actual anatomy is generated.

WO2014/139024 A1 discloses a planning, navigation and simulation system for minimally invasive therapy. The planning system allows for multiple paths to be developed from pre-operative images and scores the paths depending on desired surgical outcome of the surgery, in the particular case of brain surgery.

Known automated systems often concern a relation between the patient anatomy and either the procedural steps or the used hardware.

It is an object of the present invention to overcome the drawbacks of the prior art. In particular, the system and the method according to the present invention shall provide accurate pre-operative planning of various aspects to improve the likelihood of an optimal clinical outcome in a clinical intervention for implanting a cardiac valve.

These and other objects are solved with a system, a method and a computer program according to the independent claims.

According to the invention, a system for automated planning of a clinical intervention for implanting a cardiac valve in a patient comprises an input interface for entering data and a computing unit.

The computing unit is adapted to receive input data relating to characteristics of the patient via the input interface.

The computing unit is further adapted to automatically select a surgical tool and a surgical method characteristic based on the characteristics of the patient. The surgical tool and the surgical method characteristic are suitable for implanting the cardiac valve in the patient.

Preferably, the computing unit is adapted to coordinate the selection of surgical tool and the surgical method characteristic, i.e. to select one considering the selection of the offer.

The computing unit is further adapted to provide output data relating at least to the selected surgical tool and the selected surgical method characteristic.

Thus, the system is suitable automatically to relate characteristics of the patient to a surgical tool and to a surgical method characteristic, while proposing a procedure technique suitable for the specific patient anatomy.

Alternatively or additionally, said computing unit is adapted to receive input data relating to characteristics of the patient and to receive input data relating to a surgical tool and/or a surgical method characteristic via the input interface. The computing unit can then be adapted to automatically determine a risk value based on the characteristics of the patient and at least one of the surgical tool and the surgical method and the computing unit is further adapted to provide output data relating to the risk value.

The system may be a system for automatic planning of a trans-catheter aortic valve implantation (TAVI).

Additionally, the computing unit may be adapted to automatically select imaging tools and at least one imaging method characteristic and the computing unit may be adapted to provide output data relating to imaging tools and imaging method characteristic.

The computing unit may be adapted to provide further output, for example a ranking list of different surgical tools and surgical method characteristics, statistical data or a final report.

For example, in the context of a TAVI procedure, the computing unit can provide a list of several valve types and sizes ranked with respect to the risk of paravalvular leaks or conductivity problems after implantation.

Also, the computing unit may provide a ranking for the access site. For one patient the femoral access may be more at risk for vessel perforation than the carotid access or the inverse.

The input interface may comprise a communication interface for communication with an external device, such as an imaging device or an external expert system, and/or the input interface may allow communication with a user.

The input interface preferably is selected from the group of a keyboard, a vocal interface, a mechanical button or switch, a mouse, joystick, haptic glove, a graphical user interface, in particular a touchscreen, a motion detector, in particular a gaze tracker or a head motion detector, a virtual reality or an augmented reality interface, and a 3D monitor, in particular a hologram presentation combined with a haptic glove or a motion detection.

The computing unit may be adapted to provide output data before and/or during a partially or fully automated surgical intervention.

The risk value may relate to or may be at least partially based on reported post operation complications due to the valve, due to human errors in planning (due to obvious errors detected in the input), due to a human error in intervention (such as a chosen valve or surgical method, which is connected with a high risk for a respective patient, for example based on statistics for a patent group and/or a specific procedure), due to material other than valve, due to a specific anatomy of the patient, for example an unusual tortuous path.

The risk value may also be based on or relate to a presumed complication, e.g. based on an average duration of the procedure, for example due to information extracted from medical images, in particular due to a high number of images. A high number of images may be an indication of a difficult situation and a time-consuming procedure.

The risk value may also be based on or relate to presumed complication, e.g. based in imaging parameters during the procedure. Elaborated medical image information, such as due to the necessity of a repetitive C-arm angle change, may be in indication for complications.

The risk value may be evaluated by using training data which are based on previous interventions, which may give hints to difficulties although they have not explicitly been reported.

The training data may be based on physician's reports, saved in a data base. The physician's reports may be stored in a schematical format or may be stored as a text file or may be stored as an image file, which may be treated by OCR.

The computing unit may be adapted to determine the risk value based on at least one of biomechanical modelling and an imaging, such as US imaging of CT scan.

Generally, input data may for risk determination be taken from databases which are independent of an individual patient (model database) and databases which are specific for an individual patient (new case database).

The model database data may comprise a statistical atlas of the aortic anatomy, for example built and based on a collection of CT images, a list of implanted transcatheter heart valve types and sizes, a list of instruments used during an implantation procedure, a list of short-term and long-term outcomes.

The new case database may comprise CT images of the anatomy of a specific patient, the patient clinical history, comprising precedent conductivity disturbances, precedent strokes, arrythmia, grades of stenosis and/or other comorbidities. The new case database may also include a list of already selected tools to be used for the procedure.

The system may comprise an output interface for receiving the output data from the computing unit and for providing the output data to a user and/or to an external device.

The output interface may be selected from the group of optical or visual output interfaces, an augmented reality display, a virtual reality display, a haptic feedback device or acoustic output interfaces.

The output interface may allow to present output data such that a user may react on the output data. The output device may be part of the input interface or may be integrated in an interface device comprising the input interface.

The output interface may be connected or connectable to a surgical robot for carrying out the surgical procedure.

The input data may be selected from the group consisting of imaging data, in particular 2D and/or 3D imaging data and/or 2D sliced imaging data, characteristics relevant for access into a and closure of a vessel, characteristics relevant for pacing, characteristics relevant for imaging, characteristics relevant for navigation, characteristics relevant for valve positioning and implantation, characteristics relevant for commissural alignment, characteristics relevant for a surgical tool and/or a surgical method.

The 3D imaging data may include one or a combination of MRI (Magnetic resonance imaging)-data, CBCT (Cone-beam computed tomography)-data, multi-view ultrasound data, 3D ultrasound data and data of a surface scanning, for example using pressure sensors or a balloon.

The imaging data may directly be provided by an imaging device or supplied as preoperative images taken from a storage.

The characteristics relevant for access and closure may comprise data concerning a presence of a femoral artery disease, for example calcifications or plaques, such as extents, locations and dimensions of possible calcium depots, data concerning an existence of a prior access, data concerning distances of vessels from the skin, data concerning a presence of an aneurism and/or a thrombosis.

The characteristics relevant for pacing may comprise data concerning calcifications, data concerning a history of conduction disorders, for example when there is pre-existing pacemaker or data concerning the size and the location of the membranous septum. The characteristics relevant for pacing may also comprise data concerning the shape of the aortic root and the left ventricle. For a given shape, the pacing might be more or less efficient. For example, anatomical constraints may impose a limited contact with the pacing wire, therefore a less optimal pacing will be obtained.

The characteristics relevant for imaging, for example relevant for imaging during navigation or relevant for interpretation of entered images, may relate to (i) the aortic shape, such as aortic tortuosity, and/or may relate to (ii) the aortic state, such as the presence of an aneurism and/or a thrombosis, the existence of calcifications and/or plaques.

The characteristics relevant for imaging, may also relate to possible contrast agents, for example to a contrast agent intolerance, or to a limited access for placing an imaging device, for example due to necessity of a specific positioning of the patient. The characteristics relevant for imaging may also relate to conditions, for example anatomic conditions, which make introduction of contrast agent necessary or desirable such that more complex structures are visible.

The characteristics relevant for navigation may relate to (i) the aortic shape, such as tortuosity, diameter, shape anomalies, to (ii) the aortic state, such as presence of an aneurism and/or a thrombosis, existence of calcifications and/or plaques as well as impedance, capacity, stiffness and shape of calcifications and/or to (iii) the strain at possible contact areas with the valve.

The characteristics relevant for valve implantation may include characteristics of the native valve to be functionally replaced, for example shape, such as diameter, and state of native valve, such as kind of malfunction or grade of calcification and orientation of the commissures.

The characteristics relevant for valve implantation may include the type of the valve to be implanted and the target position and/or the target orientation of the valve to be implanted with respect to the native valve to be functionally replaced, in particular the intended implantation depth, the tilt and the orientation of the valve to be implanted.

The characteristics relevant for valve implantation may, in particular, include the shape of the left ventricular outflow tract, which may affect the depth of the implanted valve.

The characteristics relevant for valve implantation may concern shape, stress and strain at intended contact surfaces with the valve implant, a predicted conduction disorder and/or predicted leaks.

Conduction problems and leaks may appear if the implant size is not appropriate. Ideally, based on the patient anatomy, in particular based on the strain and stress at the contact surface, predictions on conduction disorder and/or leaks may be made at the planning phase. Thus, predicted conduction disorder and/or predicted leaks are second-order parameters.

The characteristics relevant for valve implantation may also relate to a risk of rupture and/or migration, which are second-order parameters and may be computed from stress and strain.

The characteristics relevant for commissural alignment may relate to the intended orientation of an implant valve at the implant position and/or to an orientation of an implant valve within a delivery device, for example to the valve crimping position, which may be adjustable and/or which has to be taken into account.

The computing unit may be adapted to select a surgical tool from the group consisting of implantation devices and valves.

The implantation devices may in particular comprise guidewires, for example robotic guidewires, introducer kits, catheters, a robotic manipulator, such as a growing robot, and/or delivery devices.

Additionally, an access tool, a closure tool and/or an imaging tool, as well as related characteristics such as type, shape, size of access and closure tool, type and size of imaging tool, may be selected.

Additionally, a preparing tool, such as dilation balloon for dilating before implanting, may be selected separately from the delivery tool.

The system proposes optimized surgical tools for a safe and efficient intravascular intervention.

The output data relating to the surgical tool may include tool characteristics selected from the group of stiffness, length, diameter, brand, model and type of a delivery device, such as a TAVI catheter, shape, diameter and/or parameters for a deployment workflow of a growing robot, shape, orientation, size, brand, model and/or type of a valve implant, size, shape, brand, model and/or type of a puncturing needle.

The orientation of the valve implant may be related to the orientation of the implant valve within the delivery device, for example with respect to a valve holder and/or to a delivery catheter.

The parameters for a deployment workflow of a growing robot may include number and timing of steps, as well as pressures to be applied.

The output data relating to the surgical method characteristic may be correlated with the output data relating to the surgical tool, since the surgical method characteristics may depend on a surgical tool and vice versa.

Similarly, the computing unit may be adapted to combine suitable tools and surgical method characteristics for minimizing the risk of the procedure. The selection of a first tool may depend on the selection of a further tool and the selection of a first surgical method characteristic may depend on the selection of a further surgical method characteristic.

The output data relating to the surgical method characteristic may comprise a simulation of the access, the navigation and/or the deployment of the valve implant, in particular of the complete clinical intervention.

The output data relating to the surgical method characteristic may include method characteristics selected from the group of
- an access site,
- information about placement of a temporary pacemaker.
- information about stent preimplantation,
- information about a path to puncture,
- information about a dilation process during the access phase, in particular information about the access port dilation process,
- information about a path to navigate,
- information about a final position of the valve implant,
- information about an optimized orientation and/or shape of the valve implant,
- deployment parameters,
- imaging instructions.

Additionally, the output data relating to the surgical method characteristic may include method characteristics concerning closure steps.

Information about the access site may include the location of an access site and a specification of an access site for a respective tool, such as a pacing access, a pigtail access, an access for stent preimplantation or a preliminary secondary access.

Typically, the position of an access site is determined and output for each tool. For example, a pigtail is inserted from a radial access site, a pacing device from a primary femoral access site or from a secondary femoral access site.

Depending on the type of the tool, a specific location of an access site may be necessary. For example, a temporary pacemaker might be required through a venal access.

The access site may be linked with parameters for indicating the access site on the patient and/or on an image.

The information about placement of a temporary pacemaker and the information about a stent preimplantation may concern the presence of a stent and/or a pacemaker, and if applicable, the type, the position of the stent and/or the access site of the stent and/or the pacemaker.

The path to puncture may consider the presence of a stent and/or calcifications. The puncture path planning should ensure a path free of a possible collision with an already implanted device, e.g. a stent. The orientation of the needle may have to be adapted. Information about the path to puncture may therefore include information about a needle orientation.

Information about the path to puncture may include parameters for control of a puncture tool.

Information about the dilation process during the access phase may be optimized considering at least one of (i) a depth of an artery, (ii) a presence of calcifications, (iii) mechanical properties of the vessel and/or (iv) shape and/or at least one mechanical property of the surgical tool.

Information about the dilatation process may include parameters for control of a dilation tool during the access phase, such as choosing or setting the size of an introducer.

The computing unit may be adapted to compute a calcification distribution and/or positions of calcifications and/or a 3D geometrical model of the access region.

The computing unit may be adapted for combining at least one of a biomedical model to predict deformation and an associated risk, computed calcification distributions, computed calcification positions and the 3D geometrical model of the patient, in particular of the access region.

The information about the path to navigate may be optimized considering the selected surgical tools and characteristics of the patient, in particular the anatomy.

The path to navigate may be optimized considering at least one of (i) calcifications, (ii) abnormal geometry of a vessel, for example due to an aneurysm or a thrombosis, (iii) shape and mechanical properties of a vessel and (iv) shape and mechanical properties of the selected surgical tool.

Preferably the computing unit is adapted to use at least one of a parametric shape model and a biomedical model to predict the deformation of vessels and/or to show locations of calcifications and plaques, which are extracted from images.

Such a model may be used for determining the path to navigate, the path to puncture as well as for planning dilation and preparing the native valve for implantation.

It may be necessary to crack and to open a calcified native valve by a dilation tool before the new valve can be placed.

Information about the path to navigate may include parameters for control of a navigation tool, such as a robotic guidewire. Information about deployment parameters may include details concerning the kind of the valve expansion, such as active or passive valve expansion, concerning the degree of valve expansion and concerning the necessity of pre-dilation and/or post-dilation.

Information about deployment parameters may include a balloon pressure for dilation of the valve implant, for pre-dilation and/or for post-dilation, if applicable.

Information about deployment parameters may include parameters for control of a deployment tool.

The imaging instructions may include imaging times and/or irradiation doses optimized according to the patient's anatomy. Fluoroscopy for example may be skipped for segments of the anatomy, which are trivial to navigate.

The imaging instructions may include instructions concerning injection of a contrast agent, in particular concerning the timing of the injection, the target region and/or the amount of the contrast agent, in order to make visible complex segments.

Information about a final position of a valve to be implanted, about the path to navigate and an optimized implant orientation and/or shape may relate to a commissural alignment, which is useful in order to facilitate coronaries access after a transcatheter heart valve implantation.

Information about the path to navigate may include information about the angle for insertion the delivery device into the vessel and/or into the implant position to orientate the implant valve in a proper way.

An optimal implant position and an optimal implant orientation allows access to the coronaries.

The output data relating to the surgical method characteristics may further include risk parameters.

Risk parameters designate factors that may be responsible for a possible risk.

Risk parameters may point to a risk of unwanted events during the clinical intervention, such as vessel perforation, vessel dissection, dislocation of calcium depots, valve embolization, leaks, conductivity problems, coronary obstructions, in particular related to a non-optimal valve position, annular ring ruptures, in particular related to pre- and post-dilation, as well as cardiac tamponades, in particular related to the manipulation of a stiff guidewire.

The risk parameter may include a combination of a shape property and mechanical properties of the patient anatomy which are related to the selected surgical tool and/or method.

The risk parameter may include an area of the patient which requires particular attention.

In the respective area there may be a risk for a plaque rupture, a dislocation of calcifications, a dissection of a vessel and/or a perforation of the vessel, in particular during navigation or during access or retraction.

The respective area may be automatically recognized on images by means of image analysis.

The image analysis may include region detection in fluoroscopic images or region detection in CT scans, as well as image fusion of CT and fluoroscopy images.

The risk of a plaque rupture may be quantified by the position, the surface stiffness and/or a contact stress. The risk of a dislocation of calcifications may be quantified by the position and the amount of calcifications. The risk of a dissection of a vessel may be quantified by a wall stiffness, a presence of calcification and/or a contact stress between a tool and a vessel wall.

A strain/stress value at a specific location, such as a wall stiffness, as well as a thickness and a location of a calcifications may be calculated on basis of the biomechanical model.

The risk parameter may include a specification of a stress and/or a strain. These quantities correspond to the risk of a rupture or a dissection.

The computing unit may be adapted to determine a stress and/or a strain based on at least one of biomechanical modelling and an imaging, such as US (ultrasound) imaging and/or a CT (computer tomography) scan.

The risk parameter may include a local and/or regional shape of an artery including tortuosity.

The respective local and/or regional shape of an artery may be automatically recognized on images by image analyses done with the computing unit.

The computing unit may be adapted to determine the local and/or regional shape of an artery based on at least one of (i) curvature measures of the centerline, for example computed from a pre-operative CT scan or a fluoroscopy with contrast agent, (ii) a surface mesh, (iii) shape parameters, in particular associated with regional artery tortuosity, (iv) determining intra-patient versus inter-patient variability and (v) identifying areas/regions of major risk.

The risk parameter may be quantified by the number of bends with respect to a reference "straight" anatomy and their respective curvature angles.

A specific combination of bends and/or a specific curvature pattern can be associated with a high risk and should be avoided during navigation.

Shape parameters may be stored as a statistical atlas in a memory which can be accessed by the computing unit.

The risk parameter may include a shape or anatomical abnormality, in particular a shape or anatomical anomaly of a vessel and/or of the native valve.

A shape or anatomical abnormality can by identified using an atlas.

A shape parameter describing the size of an aneurism in the abdominal area can for example be determined on the basis of a 3D shape of a patient. The shape parameter may be determined using a principal component analysis (PCA) on the 3D shape. By PLS (partial least square) regression the size of the aneurism may be estimated and be related to a respective PCA model.

This model can directly be applied to a new patient 3D shape to identify and extract the size of the aneurism. The same procedure can be used for other geometrical abnormalities.

The respective shape or anatomical abnormality may be automatically recognized on an image of the patient by image analysis done with the computing unit.

The computing unit may be adapted to determine a shape or anatomical abnormality based on at least one of (i) using a standard deviation from the mean population shape or anatomy or a standard deviation from a subgroup population, (ii) extracting a vessel diameter, preferably based on preop-CT, US imaging, (iii) an online estimation by contact with a guidewire, (iv) a blood flow analysis, in particular by detecting deviations from the normal flow.

The risk parameter may include a distribution and/or an amount of calcification.

The output data may also relate to a risk value as a result of a risk analysis and/or to an anticipation of a risk, in particular based on risk parameters.

The computing unit may be adapted to determine the risk parameter based on at least one of (i) pre-op CT (computer tomography) or US (ultrasound) imaging segmentation to extract a pixel-wise position and (ii) an inline stiffness estimation, preferably from sensitized impedance/capacitance of the artery wall.

The computing unit may be adapted to analyse the pre-op CT images or US images by AI (artificial intelligence) software and/or by traditional methods.

The computing unit may further be adapted to determine and to output an overall risk rate, risk probability and/or a risk map based on the risk parameters.

The risk probability is a measure of the likelihood of a risk occurring.

In the case of identified risks, the computing unit may be adapted to provide improved or alternative output data relating to the surgical method further including alternative output data, in particular an alternative path and/or an alternative device and/or a manual or surgical implantation.

The computing unit may be adapted to provide at least one parameter for controlling a robotic guide wire along the alternative path.

The computing unit may be adapted to automatically select or recommend the surgical tool and the surgical method characteristic by considering an optimized combination of one or more tools and/or of one or more method characteristics minimizing the risk of the procedure and/or maximizing the expected outcome.

A maximized outcome may be related to a highest pressure gradient caused by the implanted valve, a minimal exposure to radiation, a minimal procedure time, a maximum of flow in the replaced valve, an optimal orientation of the replaced valve.

Additionally or alternatively, the computing unit is adapted to automatically select the surgical tool and the surgical method characteristic by considering the presence of a stent and/or calcifications areas with high risk of rupture or migration.

Calcified areas may pose a risk for a rupture and therefore a risk to the entire procedure.

The computing unit may be adapted to receive user feedback via the input interface. The feedback may be a validation feedback or a change feedback.

The computing unit may be adapted to use the user feedback for planning. Depending on the feedback, the computing unit may be adapted to repeat the selection, to confirm the selection and/or to amend the selection. The feedback may induce a change of the selected path and/or the selected combination of instruments.

The user may change the automatically selected surgical tools and surgical method characteristics.

For example, in case an area to be avoided is suggested due to a risk parameter, such as a rate of calcification, resulting in a stress and strain at the interaction with a surgical tool, the user may enter a feedback by correcting by hand the path to take.

The user may choose to avoid the area to be avoided or may choose to pass this area nevertheless.

The user may also impose the use of a pacemaker.

The computing unit may be adapted to calculate a risk of the procedure with given surgical tools and given surgical method characteristics. In particular, the computing unit may be adapted to calculate a risk of the procedure with surgical tools and surgical method characteristics proposed by user feedback.

The computing unit may be adapted to create a virtual model representing a body part involved in the procedure. The virtual model may be created based on patient characteristics. The computing unit may be adapted to automatically select the surgical tool and the surgical method characteristic based on the virtual model.

The creation of the virtual model may comprise a method to morph an anatomical template to a set of points representing a body part involved in the procedure, particularly at least one of a landmark fitting and a parametric surface fitting.

The computing unit may be adapted to annotate CT (computer tomography) images, fluoroscopy images and/or US (ultrasound) images, for example pixelwise in a partly or fully automated manner.

The computing unit may be adapted to fuse a plurality of images to provide an overall image. The plurality of images may be images of the same area provided by different imaging sources. The plurality of images may also be provided by the same imaging device but with different techniques or with different projection planes or imaging angles.

The computing unit may be adapted to automatically detect or define high-risk areas as areas with constricted vessels and/or as areas with a high concentration of calcification, with a given shape of the calcified component. The computing unit may be adapted to determine risk factors with statistical methods, by correlation of an anatomical characteristic with a known clinical outcome.

Mechanical properties of a vessel, such as a stiffness, may be accounted in combination with the respective shape. For example, the distribution of the calcification in the annular ring will define the overall annular ring stiffness, which at the end may impact the selection of a valve.

The computing unit may be adapted to automatically select or recommend the surgical tool and the surgical method characteristic based on at least one database describing anatomical features, including geometrical features, in particular the shapes of a vessels, lengths of the vessels, diameters of the vessels, thicknesses of vessel walls, and/or other non-geometrical characteristics such as missing or additional typical vessels.

The patient information may be stored in a specific database structure which allows for rapid parsing, fast patient comparison and optimal information storage.

The data structure may include multiple templates taking into account the topological geometry of a patient.

For the patient topological geometry
a) a missing branch, for example deep femoral missing branch,
b) an additional artery, as for example some patients have more than one renal artery and/or
c) a brachiocephalic trunk topology may be taken into account.

The data structure may include a shape atlas for each template. The shape atlas may capture population anatomical shape variation, for example related to diameter, length, tortuosity, for a given topology.

The database may be obtained by diffeomorphic fitting of a template according to patient topology to annotations extracted from patient medical images.

The computing unit may be adapted to automatically select or recommend the surgical tool and the surgical method characteristic based on at least one of a database describing the variation of the anatomical 3D (three dimensional) shape and a database describing anatomical variations excluding shape, such as missing or additional arterial branches.

The database describing the variation of the anatomical 3D shape may be based on or may include a statistical atlas which is made based on a topology and which describes the variation of the shape for a given population.

The databases may contain multiple templates related to the patient topology and for each template a statistical atlas.

The database may include at least one representation method selected from a parametric mesh surface, landmarks and a 3D (three dimensional) representation of the body, in particular comprising 1D (one dimensional) and/or a 2D (two dimensional) surface representation.

The database describing anatomical variations excluding shape may comprise a plurality of templates describes the variations of anatomical features excluding shape for a given population.

The computing unit may be adapted to automatically select or recommend the surgical tool and the surgical method characteristic based on at least one statistical atlas, preferably a plurality of statistical atlases, describing shape variations of an anatomic feature, in particular of at least a part and preferably of the entire aorta, among the population for each template.

The statistical atlas describing the variation of the shape of an anatomical feature for a given population may comprise tortuosity and diameters, as well as shape modes related to challenging anatomies or possible complications to be expected.

Such shape modes may include a specific diameter change in a specific area which is exceptional as compared to a statistical model. Shape modes may also include challenging anatomies defined as deviating from the population mean shape by a predetermined range or they may include specific tortuosity combinations as compared to a statistical model.

The computing unit may be adapted to automatically select or recommend the surgical tool and the surgical method characteristic based on a plurality of templates describing the topological variation of the aortic anatomy.

The surgical tool or method may be selected by pure geometrical analysis, for example due to a valve selection, and/or by a statistical analysis including a comparison of the new shape to a shape atlas and/or by using a biomechanical model to predict possible outcomes like a vessel dissection or an incorrectly fitted implant.

In the context of the statistical analysis, a probability that a specific tool is not suitable for a patient may be based on the knowledge on how this tool performs in a population or ins subgroup of a population of patients with similar shapes.

The templates in particular include at least one representation method selected from a parametric mesh surface, landmarks and a 3D (three dimensional) representation of the body, in particular comprising 1D (one dimensional) landmarks and a 2D (two dimensional) surface representation.

The computing unit may be further adapted to classify the patient by a comparison of patient characteristics with a plurality of average templates.

The patient may be compared to existing patients taken into account in the database by means of the template.

The closest neighbours will be used to classify the new patient in a category. This category may be used to define the type of the surgical method or the surgical tool to be used.

The database may contain averaged data concerning characteristic data of specific areas relevant for the clinical intervention. The database may contain the patient's 3d anatomy as well as the tools having been used during a procedure and the experienced outcomes.

This information may be used to create statistical models with a good predictive power for the new incoming patients.

The computing unit may be adapted to query the database.

A suitable template may be used to create, to store and to query the database.

The computing unit may be adapted to determine a local deformation on the patient's body resulting from a force applied by an external actor to an area of the patient's body, especially by tools selected.

Biomechanical modelling may be used to determine the local deformation.

The computing unit may be adapted to determine the local deformation based on at least one of a statistical atlas of deformations, a biomechanical model considering tissues properties and anatomical constraints, a local non-rigid registration between a template and collected data, preferably online collected data.

For using a biomechanical model the geometry, the tissues properties and the applied force may be used.

Alternatively, a non-rigid registration may be driven by the observations on real-time images. Tissues biomechanical properties, such as stiffness due to artery calcification or thickness, may constrain the motion.

The determination or prediction of local deformations may be used as a condition for selecting an appropriate tool and an appropriate surgical method characteristic, for example for dilating during access or ballooning during deployment or for advancement of a delivery tool during navigation.

The local non-rigid registration may be based on at least one of (i) fitting a parametric template to a 3D representation comprising landmarks using correspondent points , wherein measured landmarks of a 3D representation are fitted to correspondent points of a parametric template, and (ii) determining a parametric model describing the correspondence between a deformation displayed in two dimensions to a deformation in a three dimensional presentation, in particular using at least one of a 3D shape model, tissue properties and anatomical constraints for transferring features from a 2D image into a 3D representation.

A two-dimensional presentation may correspond to a projected or a sliced presentation of a patient area.

The patient shape, biomechanical properties and boundary conditions, for example relating to surrounding organs, are relevant for a transformation of 2D image into a 3D image.

Assuming the 3D shape of the patient and a 'camera position' of a 2D image are known, it is possible to extract local deformation from respective 2D images.

Furthermore, based on a biomechanical model a deformation extracted from a 2D image may be translated locally in a 3D deformation.

The computing unit may be further adapted to associate characteristics of the patient, in particular a patient-specific anatomy or pathology, with geometrical and/or mechanical properties of the surgical tool.

The computing unit may be adapted to associate characteristics of a patient with properties of the surgical tool based on a statistical shape analysis combined with a partial least square regression (PLSR) to a patient's profile related to a specific design of a tool, such as a delivery tool, a catheter, or a valve.

The patient characteristics may be defined by shape, patient history and clinical parameters.

PCA can be used to reduce the number of characteristics.

A database may contain the information on which tool has been used for which 'type of patient', based on experiences of experts who did the best choice for the given patient.

PLSR may be used to define the phenotype of the patient by matching the patient characteristics with the database. Data of a new patient are compared to the database. A tool corresponding to a determined patient already existing in the database may be recommended, wherein a determined patient is chosen from the database such that the distance between the characteristics of the new patient and the characteristics of the determined patient is minimal.

The computing unit may be further adapted to associate characteristics of a patient with surgical method characteristics, for example with steering commands for navigation, commands concerning pressure to be applied during dilation, etc..

The computing unit may be adapted to associate patient's characteristics with properties of the surgical tool based on a geometrical metrics, for example characterizing the tortuosity.

The computing unit may be adapted to sequentially provide output data for each stage of the clinical intervention, in accordance with a sequence corresponding to the sequence of the clinical intervention.

The clinical intervention may comprise a plurality of steps. A stage of the clinical intervention may comprise a single or a plurality of subsequent steps of the clinical intervention.

A stage may e.g. be related to the usage of a specific tool.

A first stage may concern the preparation of the clinical intervention, wherein a suitable valve and a suitable deployment tool is to be selected. A subsequent stage may concern the access, wherein an access site and an access tool is to be selected. A subsequent stage may concern the navigation, wherein a navigation tool and/or characteristics of the navigation path and respective control parameters are to be selected. A subsequent phase may relate to valve implant positioning, to valve implant deployment and to valve implant testing for which respective control parameters are to be selected. A subsequent phase may relate to the retraction of tools and a final phase may concern the closure of the vessel, for which also a respective tool and/or respective control parameters are to be selected.

The computing unit may be adapted to sequentially provide output data for each stage during the clinical intervention before performing the respective stage or in a planning phase before the clinical intervention. The output data of a stage may also be based on information gathered during a previous stage.

The computing unit may be adapted to automatically provide a checklist.

In particular, the checklist may be provided after completion of a selection for each stage and/or before starting each stage of the clinical intervention.

According to another aspect of the invention, a method for computer based automatic planning of a clinical intervention for implanting a cardiac valve in a patient comprises the steps of (i) receiving input data relating to characteristics of the patient via an input interface, (ii) automatically selecting or recommending a surgical tool and a surgical method characteristic suitable for implanting the cardiac valve in the patient, and (iii) providing an output data relating at least to the selected or recommended surgical tool and to the surgical method characteristic. The selection is made by a computing unit based on the characteristics of the patient.

The clinical intervention may be a trans-catheter aortic valve implantation procedure.

Additionally or alternatively, input data relating to characteristics of the patient and input data relating to a surgical tool and/or a surgical method characteristic may be received via the input interface. A risk value based on the characteristics of the patient and at least one of the surgical tool and the surgical method may be determined. Output data relating to the risk value may be provided.

Preferably, a system as described above is used.

The input data may be selected from the group consisting of imaging data, in particular 2D and/or 3D imaging data, characteristics relevant for access and closure, characteristics relevant for pacing, characteristics relevant for imaging, characteristics relevant for navigation, characteristics relevant for valve positioning and implantation, characteristics relevant for commissural alignment, characteristics relevant for a surgical tool and/or a surgical method. Details of the input data are the same as explained above.

The surgical tool may be selected from the group consisting of an implantation device and a valve. Details of the selected tool are the same as explained above.

The output data relating to the surgical tool may include tool characteristics selected from the group of stiffness, length, diameter, brand, model and/or type of a delivery device, shape, diameter and/or deployment workflow a growing robot, type, shape, orientation, brand, model and/or size of a valve implant, type, size, brand, model and/or shape of a puncturing needle.

Details of the output data relating to the surgical tool are the same as explained above.

The output data relating to the surgical method characteristic include method characteristics selected from the group of
- an access site,
- information about placement of a temporary pacemaker.
- information about stent preimplantation,
- information about a path to puncture,
- information about a dilation process during the access phase,
- information about a path to navigate,
- information about a final position of the valve implant,
- information about an optimized orientation and/or shape of the valve implant,
- deployment parameters,
- imaging instructions.

Details of the output data relating to the surgical method characteristic are the same as explained above.

Preferably, the respective method characteristics are provided in the order of the procedure of the clinical intervention.

The output data relating to the surgical method characteristics may further include risk parameters as described above.

The output data relating to the surgical method characteristic (AS, VP, PS) further may include risk parameters selected from the group of areas which require particular attention and at least one of strain and stress or anatomical or pathological variation.

The risk parameter may be determined based on at least one of biomechanical modelling and an imaging, such as US (ultrasound) imaging and/or a CT (computer tomography) scan.

The risk parameter may include a local and/or regional shape of an artery including tortuosity and may be determined as explained above.

The risk parameter may include a shape or anatomical abnormality and may be determined as explained above.

The risk parameter may include a distribution and/or amount of calcification and may be determined as explained above.

The method may comprise the further step of determining and outputting an overall risk rate based on the risk parameters as described above.

In the case of identified risks, the output data relating to the surgical method may further include alternative output data, in particular an alternative path. Output date relating to a path may include parameters for by control of a robotic guidewire.

The automatic selection or recommendation of the surgical tool and the surgical method characteristic may be made taking into consideration an optimized combination of one or more tools and/or a method characteristic minimizing the risk of the procedure.

The automatic selection of the surgical tool and the surgical method characteristic may be made taking into consideration the presence of stent and/or calcifications areas with high risk of rupture or migration. Preferably, the automatic selection provides for minimizing overall and/or specific risk.

The method may further comprise receiving feedback for planning via the input user interface. Details of the feedback are the same as explained above.

The method may comprise the further step of creating a virtual model representing a body part involved in the procedure, wherein the virtual model is created based on the patient characteristics and wherein the automatic selection or recommendation is made based on the virtual model. Details concerning the virtual model are the same as explained above.

The automatic selection or recommendation may be made based on a database describing the variation of the anatomical 3D shape. The automatic selection may be made based on a plurality of templates describing the anatomical or pathologic variation excluding shape. Details concerning the automatic selection are the same as explained above.

The method may further comprise the step of classifying the patient by comparison with a plurality of average templates. Details concerning the classifying the patient are the same as explained above.

The method may further comprise the step of determining a local deformation on the patient's body resulting from a force applied. Details concerning the determining a local deformation are the same as explained above.

The method may further comprise the step of associating the characteristics of the patient, in particular a patient-specific anatomy or pathology, with geometrical and/or mechanical properties of the surgical tool. Details concerning the associating are the same as explained above.

The output data may be sequentially provided for each stage of the clinical intervention, in accordance with a sequence corresponding to the sequence of the clinical intervention. Details concerning the sequentially providing are the same as explained above.

The method may comprise the further step of automatically providing a checklist, in particular after completion of each stage of the clinical intervention. Details concerning the providing of the checklist are the same as explained above.

The invention also provides a computer program comprising program code for carrying out the steps of the method as described above when the program is executed on a computer.

The system, the method, the computer program and the computer program product may be applied in an analogue way for automatic planning of a clinical intervention for implanting a gastrointestinal implant in a patient, such as a bariatric implant, for example a gastric bypass or gastric band.

The invention also provides a computer program product which can be loaded directly into the internal memory of a digital computer and which comprises software code portions executing the method steps as described above when the program is running on a computer.

The invention is explained in the following by means of exemplary embodiments and the accompanying drawing, in which:
- Fig. 1: is a schematic presentation of a system according to the invention;
- Fig. 2: is a schematic presentation of selection criteria for a trans-catheter aortic valve implantation;
- Fig. 3: shows a view to the annular plane of an aortic root having (i) a native valve and (ii) having an implanted valve.

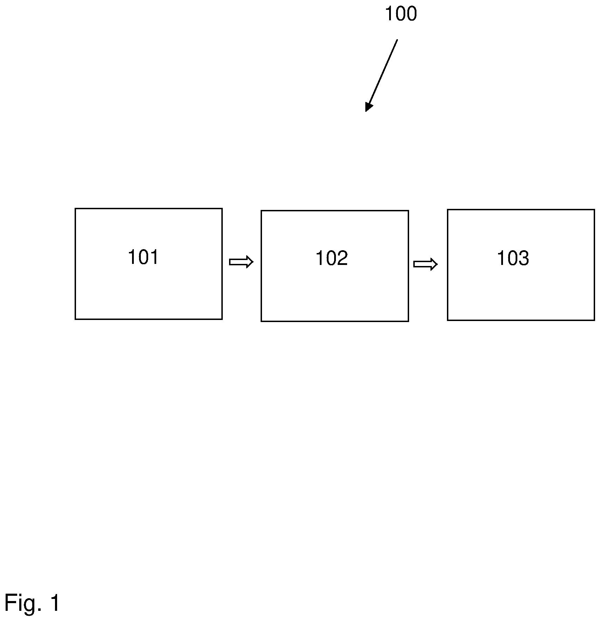

Figure 1 is a schematic presentation of a system 100 according to the invention comprising an input interface 101, a computing unit 102 and an output interface 103.

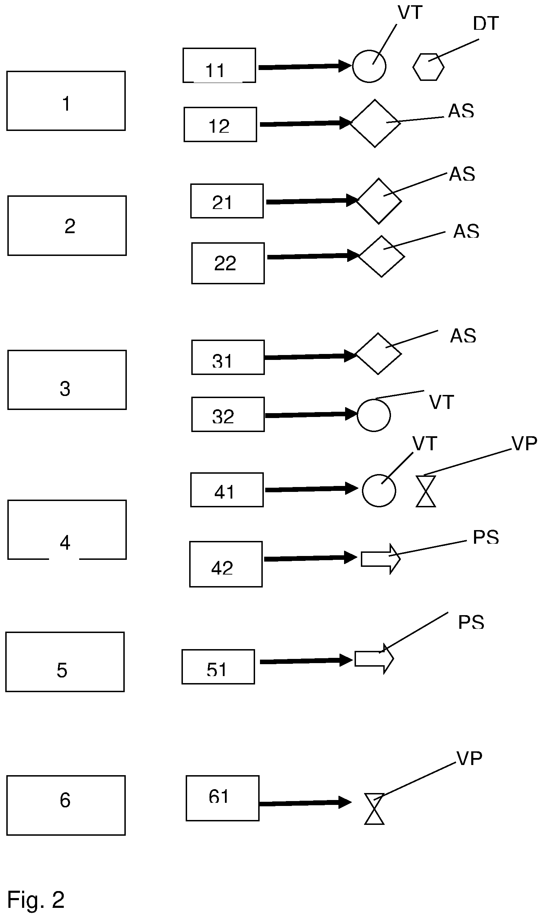

Figure 2 is a schematic presentation of selection criteria for a trans-catheter aortic valve implantation.

For planning a trans-catheter aortic valve implantation, surgical tools, at least a specific valve implant VT and a delivery device DT, and several surgical method characteristics AS, VP, PS have to be selected.

The selection may be explained with respect to relevant anatomic areas 1, 2, 3, 4, 5, 6, such as the illiaco-femoral vasculature 1, the aorta 2, the ascending aorta 3, the aortic root 4, the left ventricular outflow tract (LVOT) 5, and the membranous septum 6.

Each anatomic area 1, 2, 3, 4, 5, 6 can be characterised with quantitative characteristic 11, 21, 31, 41, 51, 61 and qualitative characteristics 12, 22, 32, 42.

The characteristics are taken into account for selecting the tools, such as the valve type VT and the delivery device DT, and the surgical method characteristics, such as the access site AS, the valve position VP and other procedural steps PS.

The illiaco-femoral vasculature 1 may be quantitatively described by the diameter 11. The diameter 11 influences the size of the delivery device DT, for example 14F or a 16F catheter. The diameter 11 also influences the valve type VT, for example the kind and size of the valve implant.

Qualitative characteristics 12 describing the illiaco-femoral vasculature 1 may include the tortuosity, the presence of calcifications, aneurisms and thrombotic appositions. Tortuosity and the location of calcifications may influence the choice of the access site AS.

The aorta 2 may be quantitatively described by the diameter 21. The diameter 21 may influence the choice of the access site AS. If the diameter is too small with respect to the diameter of the delivery device, then a secondary access site may be preferred.

Qualitative characteristics 22 describing the aorta 2 may include the presence of calcifications and the presence of plaques. These may influence the choice of the access site AS. If the femoral is too calcified, then there is an increased risk of vessel dissection. Also increased calcifications may increase the risk of complications at closure. Therefore, the clinicians may choose the less calcified region from the left and the right femoral. If both femorals are heavily calcified, a carotid access may be preferred.

The ascending aorta 3 may be quantitatively described by the diameter 21. The diameter 31 may influence the selection of the valve.

Qualitative measures 32 describing the ascending aorta 3 may include the curvature and the presence of calcifications. The valve type VT, in particular the shape of the prosthetic valve may be selected based on the curvature. Depending on the presence of calcifications a self-expanding valve implant or a valve implant which is expandable by a balloon has to be selected.

Quantitative characteristics 41 describing the aortic root 4 may include the perimeter of the aortic annulus, which may influence the selection of valve type VT, in particular the diameter of the valve implant.

Quantitative characteristics 41 describing the aortic root 4 may further include the annulus angulation which may influence the selection of valve type VT. Depending on the annulus angulation, a self-expanding valve implant or a valve implant which is expandable by a balloon may be selected. The annulus angulation may also influence the selection of the valve implant position VP.

Quantitative characteristics 41 describing the aortic root 4 may relate to the coronaries, for example the height with respect to the aortic annulus and the SoV (sinus of valsalva, an abnormal dilatation of the aortic root located between the aortic valve annulus and the sinotubular junction) and the diameter of the SoV. These quantitative characteristics 41 may have an impact on the choice of the valve type VT, in particular on the choice of the shape of the prosthetic valve.

Qualitative characteristics 42 describing the aortic root 4 may further include the presence of calcifications, which may be decisive on the choice of parameters for procedural steps PS, such as balloon dilation and carotid protection.

The left ventricular outflow tract 5 may be quantitatively characterised by the LVOT perimeter 51, which can have an impact on the choice of parameters for procedural steps PS such as pressure for delivery. The left ventricular outflow tract 5 may have a trapezoidal or a parallelogram shape, which effects the implantation depth.

The membranous septum 6 may be quantitatively characterised by the height 61 which influences the selection of the valve position VP.

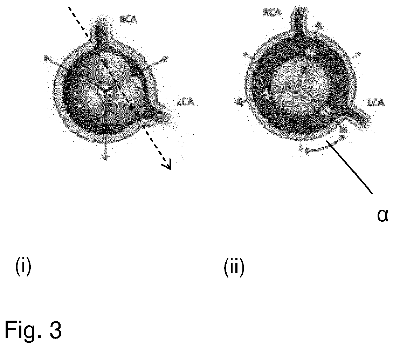

Fig. 3 shows the annular plane of an aortic root heart having (i) a native valve and (ii) having an implanted valve.

The commissures of the implanted valve may have an orientation that deviates by an angle α from the orientation of the commissures of the native valve.

As long as the deviation angle α is between 0° and 15°, "commissural alignment" is assumed. The path to the right coronary artery (RCA) and to the left coronary artery (LCA) remains undisturbed.

A "mild" commissural misalignment is assumed for an angle range between 15° and 30°, a "moderate" commissural misalignment is assumed for an angle range between 30° and 45° and a "severe" commissural misalignment is assumed for an angle range between 45° and 60°.

Due to commissural misalignment the coronary access may be limited, and consequently the coronary artery flow may be disturbed.

There may arise a stress and a strain to the leaflets, and in the worst case a central leak.

The effectiveness of the outer sealing skirt may be affected due to misalignment, and it might be necessary to reimplant the valve.

The grade of commissural alignment or misalignment can be detected by a fluoroscopic image.

In a view, which brings neighbouring cusps to overlap (see dashed arrow), one of the TAVI-valve commissural posts should be lateralized at the right side of the fluoroscopic image.

Alternatively, a view which brings the right and the left coronary ostia to overlap may be used.

The fluoroscopic image can be used to determine the eccentricity of the coronaries with respect to the commissures of the native valve.

For automatically placing an implant valve and for computing an implant position which facilitates the coronaries access after transcatheter heart valve implantation, a valve crimping position and a delivery device orientation before implantation should be determined.

A 3D geometrical model of the, preferably patient specific, aortic anatomy from femoral access to the annular ring, in particular a rigid model can be used as in input data.

Optionally, a 3D biomechanical model of the patient artery can be taken into account as further input data, which for example provides the individual eccentricity of the coronaries with respect to the native valve and which may be provided by a fluoroscopy imaging method.

A reduced model of the delivery device can be used as input data.

A model simulating the interaction of the delivery device with the static 3D anatomy or/and a model simulating the interaction of the delivery device with the dynamic 3D anatomy can be provided by the computing unit (see figure 1).

According to the intended final position within the computing unit may provide an output on how to crimp the valve on the delivery device and at which angle the delivery device should be inserted into the artery and into the native valve.

An optimal implant position relative to the coronaries position and/or relative to commissural position in the native valve may be achieved, wherein the optimal access to the coronaries after transcatheter heart valve implantation is obtained.

## Claims

1. A system for automatic planning of a clinical intervention for implanting a cardiac valve in a patient, the system (100) comprising
- an input interface (101) for entering data
- a computing unit (102),
wherein said computing unit (102) is adapted
- to receive input data relating to characteristics of the patient (1, 2, 3, 4, 5, 6) via the input interface (101),
- to automatically select a surgical tool (VT, DT) and a surgical method characteristic (AS, VP, PS) based on the characteristics of the patient, the surgical tool (VT, DT) and the surgical method characteristic (AS, VP, PS) being suitable for implanting the cardiac valve in the patient,
- to provide output data relating at least to the selected surgical tool (VT, DT) and the selected surgical method characteristic (AS, VP, PS) and/or
wherein said computing unit (102) is adapted
- to receive input data relating to characteristics of the patient (1, 2, 3, 4, 5, 6) and to receive input data relating to a surgical tool (VT, DT) and/or a surgical method characteristic (AS, VP, PS) via the input interface (101),
- to automatically determine a risk value based on the characteristics of the patient and at least one of the surgical tool (VT, DT) and the surgical method,
- to provide output data relating to the risk value.

2. A system according to claim 1, wherein the system comprises an output interface (103) for receiving output data from the computing unit (102) and for providing the output data to a user.

3. A system according to claim 1 or 2, wherein the input data are selected from the group consisting of
- 3D imaging data,
- characteristics relevant for access and closure,
- characteristics relevant for pacing,
- characteristics relevant for imaging,
- characteristics relevant for navigation,
- characteristics relevant for valve positioning and implantation,
- characteristics relevant for commissural alignment,
- characteristics relevant for a surgical tool (VT, DT) and/or a surgical method.

4. A system according to claim 1 to 3, wherein the surgical tool (VT, DT) is selected from the group consisting of
- implantation devices, in particular guidewires, a growing robot and/or delivery devices,
- valves.

5. A system to claim 4, wherein the output data relating to the surgical tool include tool characteristics selected from the group of
- stiffness, length, diameter, brand, model and/or type of a delivery device,
- shape, diameter and/or deployment workflow a growing robot, the deployment workflow including for example steps, timing of steps, setting of pressure,
- shape, size, orientation, brand, model and/or type of a valve implant,
- size, shape, brand, model and/or type of a puncturing needle.

6. A system according to one of the claims 1 to 5, wherein the output data relating to the surgical method characteristic (AS, VP, PS) include method characteristics selected from the group of
- an access site,
- information about a placement of a temporary pacemaker,
- information about a stent preimplantation,
- information about a path to puncture,
- information about a dilatation process during the access phase,
- information about a final position of a valve to be implanted,
- information about a path to navigate,
- an optimized implant orientation and/or shape,
- deployment parameters,
- imaging instructions.

7. A system according to one of the claims 1 to 6, wherein the output data relating to the surgical method characteristics (AS, VP, PS) include risk parameters, in particular areas of the patient which require particular attention.

8. A system according to claim 7, wherein the computing unit (102) is adapted to determine the risk parameter based on at least one of biomechanical modelling and an imaging, such as US imaging of CT scan.

9. A system according to claim 7 and claim 8, wherein the risk parameter is a local and/or regional shape of an artery including tortuosity and
the computing unit (102) is adapted to determine the risk parameter based on at least one of
(i) curvature measures of the centerline computed from pre-operative CT scan or fluoroscopy with contrast agent,
(ii) a surface mesh,
(iii) shape parameters associated with regional artery tortuosity,
(iv) determining intra-patient versus inter-patient variability and
(v) identifying areas/regions of major risk.

10. A system according to claim 7 to claim 9, wherein the risk parameter is a shape or anatomical abnormality and the computing unit (102) is adapted to determine the shape or anatomical abnormality based on at least one of
(i) a standard deviation from the mean population shape or anatomy or a standard deviation from a subgroup population shape or anatomy,
(ii) extracting a vessel diameter, preferably based on preop-CT or US imaging,
(iii) an online estimation by contact with a guidewire, (iv) a blood flow analysis, in particular by detected deviations from the normal flow.

11. A system according to one of the claims 7 to 10, wherein the risk parameter is a distribution and/or amount of calcification
and the computing unit (102) is adapted to determine the distribution and/or amount of calcification based on at least one of
(i) pre-op CT or US imaging segmentation to extract pixel-wise position and
(ii) an inline stiffness estimation, preferably from sensitized impedance/capacitance of the artery wall.

12. A system according to one of claims 7 to 11, wherein the computing unit (102) is further adapted to determine and to output an overall risk rate, risk probability and/or a risk map based on the risk parameters.

13. A system according to one of the claims 7 to 12, wherein in the case of identified risks, the computing unit (102) is adapted to provide output data relating to the surgical method characteristic (AS, VP, PS) further including alternative output data, in particular an alternative path and/or an alternative device and/or a manual or surgical implantation.

14. A system according to one of the claims 1 to 13, wherein the computing unit (102) is adapted to automatically select or recommend the surgical tool or the method characteristics by considering at least one of
- an optimized combination of one or more tools and/or method characteristic minimizing the risk of the procedure and/or maximizing the expected outcome;
- the presence of stent and/or calcifications areas with high risk of rupture or migration.

15. A system according to one of the claims 1 to 14, wherein the computing unit (102) is adapted to receive user feedback for planning via the input interface.

16. A system according to one of the claims 1 to 15, wherein the computing unit (102) is adapted to create a virtual model representing a body part involved in the procedure, wherein the virtual model is created based on the patient characteristics and wherein the computing unit is adapted to automatically select or recommend the surgical tool and the surgical method characteristic based on the virtual model,
the creation of the virtual model preferably comprising a method to morph an anatomical template to a set of points representing the body part involved in the procedure, particularly at least one of landmark fitting and parametric surface fitting.

17. A system according to one of the claims 1 to 15, wherein the computing unit (102) is adapted to automatically select or recommend the surgical tool (VT, DT) and the surgical method characteristic (AS, VP, PS) based on at least one of
- a database describing the variation of the anatomical 3D shape, in particular including at least one representation method selected from a parametric mesh surface, landmarks and volume,
- a plurality of templates describing the anatomical or pathological variation excluding shape and optionally at least one, preferably a plurality of statistical atlases describing shape variation of an anatomic feature, in particular at least a part and preferably of the entire aorta, among the population or a population subgroup for each template.

18. A system according to one of the claims 1 to 17, wherein the computing unit (102) is further adapted to classify the patient by comparison with a plurality of average templates.

19. A system according to one of the claims 1 to 18, wherein the computing unit (102) is further adapted to determine a local deformation on the patient's body resulting from a force applied by an external actor to an area of the patient's body, in particular based on at least one of
- a statistical atlas of deformation,
- a biomechanical model considering tissues properties and anatomical constraints.
- local non-rigid registration between template and online collected data, preferably based on at least one of (i) fitting a parametric template to 3D landmarks using correspondent points and (ii) determining a parametric model describing the correspondence between 2D deformation to a 3D deformation, in particular using at least one of a 3D shape model, tissues properties and anatomical constraints.

20. A system according to one of the claims 1 to 19, wherein the computing unit (102) is further adapted to associate the characteristics of the patient, in particular a patient-specific anatomy or pathology, with geometrical and/or mechanical properties of the surgical tool, preferably based on at least one of
- statistical shape analysis combined with partial least square regression (PLSR) to patient's profile related to a specific design of the delivery tools, catheter, or valve,
- geometrical metrics.

21. A system according to one of the claims 1 to 20, wherein the computing unit (102) is further adapted to sequentially provide output data for each stage of the clinical intervention, in accordance with a sequence corresponding to the sequence of the clinical intervention.

22. A system according to one of the claims 1 to 21, the computing unit (102) is further adapted to automatically provide a checklist, in particular after completion of each stage of the clinical intervention.

23. A method for computer based automatic planning of a clinical intervention for implanting a cardiac valve in a patient, preferably using a system according to one of claims 1-22, the method comprising the steps of
- receiving input data relating to characteristics of the patient via an input interface (101),
- automatically selecting or recommending a surgical tool (VT, DT) and a surgical method characteristic (AS, VP, PS) suitable for implanting the cardiac valve in the patient, the selection being made by a computing unit based on the characteristics of the patient,
- providing an output data relating at least to the selected or recommended surgical tool (VT, DT) and surgical method characteristic (AS, VP, PS);
and/or
- receiving input data relating to characteristics of the patient (1, 2, 3, 4, 5, 6) and to receive input data relating to a surgical tool (VT, DT) and/or a surgical method characteristic (AS, VP, PS) via the input interface (101),
- automatically determining a risk value based on the characteristics of the patient and at least one of the surgical tool (VT, DT) and the surgical method,
- providing output data relating to the risk value.

24. A method according to claim 23, wherein the input data are selected from the group consisting of
- 2D and/or 3D imaging data,
- characteristics relevant for access and closure,
- characteristics relevant for pacing,
- characteristics relevant for imaging,
- characteristics relevant for navigation,
- characteristics relevant for valve positioning and implantation,
- characteristics relevant for commissural alignment,
- characteristics relevant for a surgical tool (VT, DT) and/or a surgical method.

25. A method according to claim 23 or 24, wherein the surgical tool (VT, DT) is selected from the group consisting of
- implantation devices, in particular guidewires, a growing robot and/or delivery devices,
- valves.

26. A method according to claim 24, wherein the output data relating to the surgical tool include tool characteristics selected from the group of
- stiffness, length, diameter of a delivery device,
- shape, diameter and/or deployment workflow a growing robot, the deployment workflow including for example steps, timing of steps, setting of pressure,
- shape, size, orientation, brand, model and/or type of a valve implant,
- size, shape, brand, model and/or type of a puncturing needle.

27. A method according to one of the claims 23 to 26, wherein the output data relating to the surgical method characteristic (AS, VP, PS) include method characteristics selected from the group of
- an access site,
- information about a placement of a temporary pacemaker
- information about a stent preimplantation,
- information about a path to puncture,
- information about a dilatation process during the access phase,
- information about a final position of a valve to be implanted,
- information about a path to navigate,
- an optimized implant orientation and/or shape,
- deployment parameters,
- imaging instructions.

28. A method according to one of the claims 23 to 27, wherein the output data relating to the surgical method characteristic (AS, VP, PS) further include risk parameters, in particular selected from the group of
- areas which require particular attention,
- at least one of strain and stress and anatomical and pathological variation.

29. A method according to claim 28, wherein the risk parameter is determined based on at least one of biomechanical modelling and US imaging.

30. A method according to claim 28 or 29, wherein the risk parameter is a local and/or regional shape of an artery including tortuosity and
is determined based on at least one of
(i) using curvature measures of the centerline computed from pre-operative CT scans or fluoroscopy with contrast agent,
(ii) using a surface mesh,
(iii) using shape parameters associated with regional artery tortuosity,
(iv) determining intra-patient versus inter-patient variability and
(v) identifying areas/regions of major risk.

31. A method according to one of the claims 28 to 30, wherein the risk parameter is a shape or anatomical abnormality and is determined based on at least one of
(i) using standard deviation from the mean population shape or anatomy or from a subgroup population,
(ii) extracting a vessel diameter, preferably based on preop-CT or US imaging,
(iii) an online estimation by contact with a guidewire,
(iv) blood flow analysis, in particular by detecting deviations from the normal flow.

32. A method according to one of the claims 28 to 31, wherein the risk parameter is a distribution and/or amount of calcification
and is determined based on at least one of
(i) pre-op CT or US imaging segmentation to extract pixel-wise position and
(ii) an inline stiffness estimation, preferably from sensitized impedance/capacitance of the artery wall.

33. A method according to one of the claims 28 to 32, comprising the further step of determining and outputting an overall risk rate based on the risk parameters.

34. A method according to one of the claims 28 to 33, wherein, in the case of identified risks, the output data relating to the surgical method characteristics (AS, VP, PS) further include alternative output data, in particular an alternative path.

35. A method according to one of the claims 23 to 34, wherein the automatic selection is made taking into consideration at least one of
- an optimized combination of one or more tools and/or method characteristic minimizing the risk of the procedure,
- the presence of stent and/or calcifications areas with high risk of rupture or migration.

36. A method according to one of the claims 23 to 35, the method further comprising receiving via the input interface user feedback for planning.

37. A method according to one of the claims 23 to 36, the method comprising the further step of creating a virtual model representing a body part involved in the procedure, wherein the virtual model is created based on the patient characteristics and wherein the automatic selection or recommendation is made based on the virtual model.

38. A method according to one of the claims 23 to 37, wherein the automatic selection or recommendation is made based on at least one of
- a database describing the variation of the anatomical or pathological 3D shape, in particular including at least one representation method selected from a parametric mesh surface, landmarks and volume,
- a plurality of templates describing the anatomical variation excluding shape and optionally at least one, preferably a plurality of statistical atlases describing shape variation of an anatomic feature, in particular at least a part and preferably of the entire aorta, among the population or a population subgroup for each template.

39. A method according to one of the claims 23 to 38, the method further comprising the step of classifying the patient by comparison with a plurality of average templates.

40. A method according to one of the claims 23 to 39, the method further comprising the step of determining a local deformation on the patient's body resulting from a force applied by an external actor to an area of the patient's body, in particular based on at least one of
- a statistical atlas of deformation,
- a biomechanical model considering tissues properties and anatomical constraints.
- local non-rigid registration between template and online collected data, preferably based on at least one of (i) fitting a parametric template to 3D landmarks using correspondent points and (ii) determining a parametric model describing the correspondence between 2D deformation to a 3D deformation, in particular using at least one of a 3D shape model, tissues properties and anatomical constraints.

41. A method according to one of the claims 23 to 40, the method further comprising the step of associating the characteristics of the patient, in particular a patient-specific anatomy or pathology, with geometrical and/or mechanical properties of the surgical tool, preferably based on at least one of
- Statistical shape analysis combined with partial least square regression (PLSR) to patient's profile related to a specific design of the delivery tools, catheter, or valve.
- geometrical metrics.

42. A method according to one of the claims 23 to 41, wherein output data are sequentially provided for each stage of the clinical intervention, in accordance with a sequence corresponding to the sequence of the clinical intervention.

43. A method according to one of the claims 23 to 42, the method comprising the further step of automatically providing a checklist, in particular after completion of each stage of the clinical intervention.

44. A computer program comprising program code for carrying out the steps of the method according to any one of the claims 23 to 43 when the program is executed on a computer.

45. A computer program product which can be loaded directly into an internal memory of a digital computer and which comprises software code portions executing the method steps of at least one of the claims 23 to 43 when the program is running on the digital computer.
